# EUROPEAN PATENT APPLICATION

(11) **EP 4 316 840 A1**
(43) Date of publication of application: **07.02.2024**
(21) Application number: 22306178.9
(22) Date of filing: 03.08.2022
(51) Int. Cl.: B33Y 70/00, B29C 64/106, C12M 1/00, C12M 3/00, C12M 1/26

(54) **STERILE ENCLOSURE AND A METHOD OF PRINTING AND CULTURING A BIOLOGICAL THREE-DIMENSIONAL STRUCTURE**

(71) Applicant: SARTORIUS STEDIM FMT SAS, 13400 Aubagne (FR); UNIVERSITE CLAUDE BERNARD - LYON 1, 69100 Villeurbanne (FR); Ecole Superieure de Chimie, Physique, Electronique de Lyon, 69616 Villeurbanne Cedex (FR); Institut National des Sciences Appliquees de Lyon (Insa Lyon), 69100 Villeurbanne (FR); Centre National de la Recherche Scientifique (CNRS), 75016 Paris (FR)
(72) Inventor: Gay, Isabelle, 13124, Peypin (FR); Barbaroux, Magali, 13112, La Destrousse (FR); Dufour, Alexandre, 69006, Lyon (FR); Marquette, Christophe, 69100, Villeurbanne (FR); Petiot, Emma, 69100, Villeurbanne (FR)
(74) Representative: Derambure Conseil

(57) **Abstract**

The invention concerns a sterile enclosure (2) and a method of printing and culturing a three-dimensional biological structure in the sterile enclosure. The sterile enclosure comprising:
- a base wall (10),
- a flexible side wall (8); the side wall forming with the base wall (10) a flexible casing (12) delimiting an interior chamber (14),
- a fluid inlet port (22) and a fluid outlet port (24) which allow the circulation of a culture media,
- a needle adapter (4) fixed to the flexible side wall (8), the needle adapter (4) holding a printing needle (6),
the flexible casing protecting a biological three-dimensional structure against contamination and authorizing at the same time the move of the needle adapter (4) in three directions during a printing of a biological three-dimensional structure in the interior chamber.

## Description

### Field of the invention

The present invention relates to a sterile enclosure and a method of printing and culturing a biological three-dimensional structure.

### Technical background

It is known to print out biological three-dimensional structures onto a printing platform of a three-dimensional printers. After the structure is formed, the printed components are immersed in a media for a cell culture process. So once printed, the biological three- dimensional structure must be transferred to a separate bioreactor for the cell culture process. However, it is difficult to maintain the sterile conditions during the transfer of the biological three-dimensional structure to the bioreactor.

To avoid this difficulty, it is also known to print out the biological three-dimensional structure in a container adapted to contain and control a culture media and which also comprises a printer or a movable robotic arm equipped with a printing head. After printing, it is no more necessary to transfer the biological three-dimensional structure in a separate bioreactor for the cell culture process.

A drawback of this operating mode is that the container is cumbersome and heavy since it must contain the printer or the movable robotic arm. As a consequence, when the biological three-dimensional structure has been formed, the printer or the movable robotic arm equipped with the printing head cannot be used to print out another structure because the container must be kept hermetically closed during the cell culture process. Due to its size and its mechanical part which are greased, it can be problematic to sterilise the printer or the movable robotic arm and to keep it sterilized. As the container is large and cumbersome, it can be hard to move it to place it in another room during the cell culture process.

### Object and summary of the invention

A first purpose of the present invention is to propose a sterile enclosure which allows an easy displacement of the printing head in at least two perpendicular directions, and in particular in three perpendicular directions without generating stress on the needle and without risk of bending or breaking of the needle. As a consequence, the shape of the biological three-dimensional structure can be better controlled. Another consequence is that the printing platform does not need to be movable along a vertical direction because the sterile enclosure allows an easy move of the printing head along this direction. Accordingly, a less complex printer can be used. In the same way, the printer can use a smaller motor because there less resistance is applied to the robotic arm.

A second purpose of the present invention is to propose a sterile enclosure that is less bulky, cumbersome and heavy. Since the printer or a part of it is not comprised in the sterile enclosure, its size and its weight are significantly reduced.

A third purpose of the present invention is to propose a sterile enclosure that can be easily moved and stored after the printing process.

A fourth purpose of the present invention is to propose a sterile enclosure which allows immediate reuse of the printer after a biological three-dimensional structure has been formed.

A fifth purpose of the present invention is to propose a sterile enclosure which simplifies both the printing of a biological three-dimensional structure and its culture in a single container.

A sixth purpose of the present invention is to propose a method of printing and culturing a three-dimensional biological structure that ensures that there will be no contamination of the printed biological three-dimensional structure with the bioink remaining in the needle after printing.

These purposes have been reached by a sterile enclosure and a method as defined in the independent claims. Secondary features of the sterile enclosure are set out in the dependent claims.

### Brief description of figures

Figure 1 is a perspective and schematic view of a sterile enclosure according to a first embodiment of the present invention;
Figure 2 is a perspective and schematic view of a sterile enclosure according to a second embodiment of the present invention;
Figure 3 is a perspective and schematic view of a sterile enclosure according to a third embodiment of the present invention;
Figure 4 is a perspective and schematic view of a sterile enclosure according to a fourth embodiment of the present invention;
Figure 5 is a perspective and schematic view of a sterile enclosure according to a fifth embodiment of the present invention;
Figure 6 is a perspective and schematic view of a sterile enclosure according to a sixth embodiment of the present invention;
Figure 7 is a flowchart representing the steps of a method according to the present invention.

### Detailed description of the invention

The sterile enclosure 2 according to the invention is intended to be fixed to a printing cartridge or a barrel of a printing syringe for the printing of a biological three-dimensional structure in the sterile enclosure.

The printing syringe or the printing cartridge may comprise a bioink composition. A bioink composition is a biomaterial capable of forming a hydrogel and a cell population in a controlled three-dimensional shape. An example of bioink is described in WO 2017115056.

Preferably, for the printing operation, the printing syringe or cartridge is mounted on a displaceable printer for example on a displaceable robotic arm. The printing can also be done manually with a printing nozzle.

In the below description, the term "printing" means making a manual deposit or injection of a bioink or making a 3D-printing with a displaceable robotic arm.

In the below description, the term "flexible enclosure" will be used both for enclosures which are entirely flexible and for enclosures having a lower rigid part and which are "at least partially flexible enclosure".

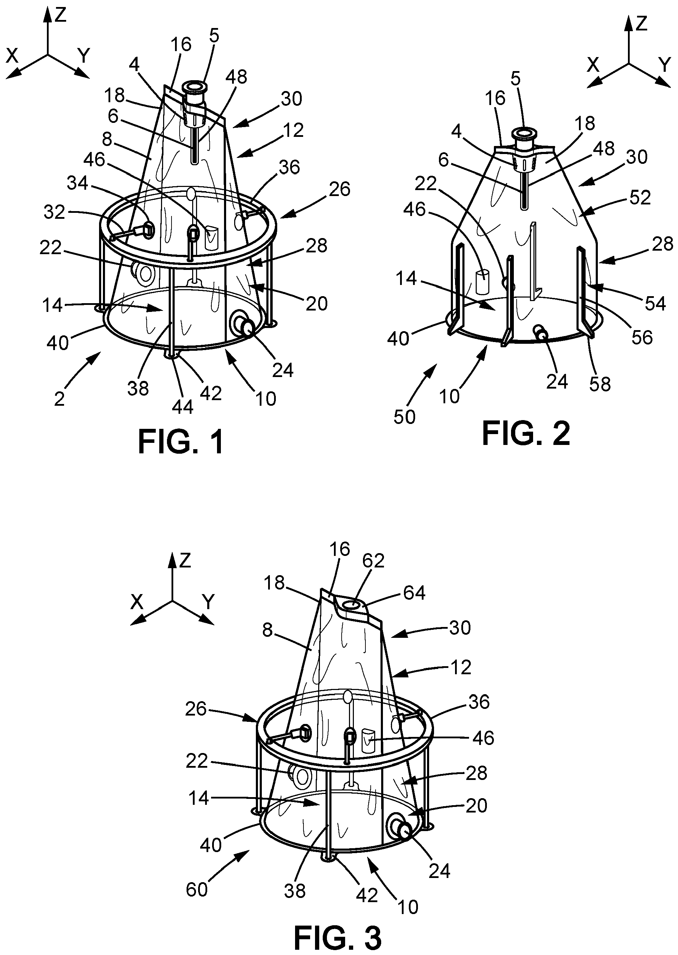

Referring to FIG. 1, the sterile enclosure 2 according to a first embodiment of the present invention comprises a needle adapter 4, a printing needle 6 held by the needle adapter, a flexible side wall 8 tightly fixed to the needle adapter 4 and a flexible base wall 10.

The needle adapter 4 here used is the tip that is usually connected to the open end of a barrel of a medical syringe. The needle adapter comprises a needle hub holding the needle and a connector part configured to be connected to a corresponding connector part of the barrel of the syringe.

The needle adapter comprises a connector part 5 conform to be connected to a complementary connector part of a printing cartridge or a printing syringe. The connector part and the corresponding connector part are for example a Luer lock connector or another aseptic connector like the connector Opta^{®} commercialised by Sartorius.

The side wall 8 forms with the base wall 10 a flexible casing 12 hermetically closed. This flexible casing 12 delimits an interior chamber 14.

In the illustrated embodiment, the needle adapter 4 is disposed on the top of the at least partially flexible casing 12.

Alternatively, the needle adapter 4 is a needle adapter which is integral with the printing cartridge or the printing syringe.

Preferably, the printing needle 6 is a biological printing needle. The needle adapter 4 and the printing needle 6 constitute a printing head.

In the illustrated embodiment, the flexible base wall and the flexible side wall can be made from one material among a thermoplastic elastomer and a thermoset.

In particular, the flexible base wall 10 and the flexible side wall 8 can be composed of a multilayer film comprising a contact layer which is in contact with the medium that fills the container, a barrier layer and an outer layer which is in contact with the external environment. The three layers are connected one to each other with a tie layer. If the container is to be filled with a biopharmaceutical product, the contact layer should be made from a material that can be in contact with this biopharmaceutical product without causing degradation of the film and of the biopharmaceutical product. Furthermore, it must be sealable on itself. For that purpose, the material is generally selected from polyolefins, such as polyethylene. The barrier layer provides a barrier to the passage of gases such as oxygen, carbon dioxide and is typically made from ethylene vinyl alcohol (EVOH).

The outer layer contributes to the mechanical strength of the wall. For that purpose, it must be sufficiently flexible to withstand high mechanical stress but not be too much stretchable in order to prevent deformation of the enclosure when it is filled with a product. The outer layer could comprise a polyolefin alone or in mixture with a copolymer of ethylene and a-olefin. A multilayer film as described in EP 20945799 could for example be used.

The flexible side wall 8 can be made from a unique sheet, for instance, when the flexible casing has a cylindrical shape. The side wall 8 of the flexible casing12 can also be made from several sheet for instance when the flexible casing has a parallelepiped shape. In this patent application, the term "a side wall" will be used to designate these two possibilities in order to simplify the description.

The base wall 10 can also be made from one or several sheet(s).

Alternatively, the base wall and the side wall can be made in silicone.

Preferably, the silicone can be one silicone among an unsaturated rubbers that can be cured by sulfur vulcanization, a Natural polyisoprene: cis-1,4-polyisoprene natural rubber (NR) and trans-1,4-polyisoprene gutta-percha, a Synthetic polyisoprene (IR for isoprene rubber), a Polybutadiene (BR for butadiene rubber), a Chloroprene rubber (CR), a polychloroprene, a Neoprene, a Baypren etc., a Butyl rubber (copolymer of isobutene and isoprene, IIR) Halogenated butyl rubbers (chloro butyl rubber: CIIR; bromo butyl rubber: BIIR), a Styrene-butadiene rubber (copolymer of styrene and butadiene, SBR) a Nitrile rubber (copolymer of butadiene and acrylonitrile, NBR) also called Buna N rubbers, a Hydrogenated nitrile rubbers (HNBR) Therban and Zetpol, (Unsaturated rubbers can also be cured by non-sulfur vulcanization if desired.), a Saturated rubbers that cannot be cured by sulfur vulcanization, EPM (ethylene propylene rubber, a copolymer of ethene and propene) and an EPDM rubber (ethylene propylene diene rubber, a terpolymer of ethylene, propylene and a diene-component), an Epichlorohydrin rubber (ECO), a Polyacrylic rubber (ACM, ABR), a Silicone rubber (SI, Q, VMQ), a Fluorosilicone rubber (FVMQ), a Fluoroelastomers (FKM, and FEPM) Viton, a Tecnoflon, Fluorel, an Aflas and Dai-El, a Perfluoroelastomers (FFKM) Tecnoflon PFR, a Kalrez, a Chemraz, a Perlast, Polyether block amides (PEBA), a Chlorosulfonated polyethylene (CSM), (Hypalon) an Ethylene-vinyl acetate (EVA) and a Thermoplastic elastomers (TPE).

The flexible casing 12 has here a general flare shape. In particular, in the illustrated embodiment, the needle adapter 4 is fixed between two junctions 16 of an upper end edge 18 of the side wall. At each junction 16, a part of the end edge 18 of the flexible side wall is fixed to another part of the end edge 18 of the flexible wall to make a gusset 20.

Alternatively, the flexible casing 12 of the sterile enclosure may have a cup shape, a belt shape, a tube shape or a folding tube shape.

The sterile enclosure 2 comprises further one fluid inlet port 22 and one fluid outlet port 24 which allow the circulation of a culture media during the cell/culture process. The fluid inlet port 22 and the fluid outlet port 24 are provided with a tri-clamp system or a hose-bard connection.

Advantageously, the sterile enclosure 2 can comprise supplementary ports which are not illustrated on the figures. The supplementary ports are used for fixing sensors for controlling different parameters of the culture media like for example its temperature or its pH.

Preferably, the sterile enclosure 2 comprises a support armature 26 holding a lower part 28 of the flexible casing to keep the lower part of the side wall 8 away from the interior face of the base wall 10. Accordingly, the lower part 28 of the flexible casing cannot touch or fall in the printed three-dimensional biological structure laid down on the base wall and damage it.

Advantageously, the support armature 26 holds only the lower part 28 of the flexible casing 12.

Advantageously, the upper part 30 of the flexible casing authorizes the displacement of the printing needle 6 along a vertical direction Z while keeping the sterility of the three-dimensional biological structure during the printing process. As consequence, it no more necessary to use a 3D-printer with a vertically movable printing plate-form. Advantageously, the upper part 30 of flexible casing authorizes the displacement of the printing needle 6 in two horizontal and perpendicular directions X, Y with respect to a non-represented printing-platform while keeping the sterility of the three-dimensional biological structure during the printing process.

The sterility of the interior chamber 14 is kept during the move of the printing syringe Thus, the printing process does not need to be performed under a laminar flow hood or biological safety cabinets.

In the embodiment illustrated on figure 1, the support armature 26 is outside and spaced apart from the flexible casing 12. The sterile enclosure 2 comprises attachment links 32 fixed to the support armature 26 and to the side wall 8 at attachment points 34 to keep the lower part 28 of the flexible side wall 8 at distance from the interior face of the base wall 10. Preferably, attachment points 34 are regularly spaced around the flexible casing 12. The lower part 28 of the flexible casing is situated under the attachment points 34. The upper part 30 of the flexible casing is situated above the attachment points 34.

In the illustrated embodiment, the support armature 26 comprises a frame 36 and posts 38 supporting the frame. The frame 36 surrounds the flexible casing. In the illustrated and non-limiting embodiment, the frame 36 has an annular shape. Alternatively, the frame can have a rectangular or a square shape.

Advantageously, the posts 38 are regularly spaced around the flexible casing 12. An internal face of an edge of the flexible side wall 8 is fixed to the internal face of a peripheral edge of the base wall 10 to form an extension 40.

The extension 40 protrudes from the base wall 10 in a horizontal plane. Alternatively, the extension 40 is made from a supplementary round flexible sheet or film welded to the external face of the base wall 10. In this case, the edges of the flexible film form the extension.

In the illustrated embodiment, the extension 40 is provided with perforated tabs 42 regularly spaced around the round sheet.

Advantageously, the lower end of the posts 38 of the support armature are positioned in the holes 44 of the tabs 42. So advantageously, the extension and the base wall 10 is kept extended and flat by the support armature.

Advantageously, the extension 40 also stabilizes the positioning of the flexible casing on a support.

The extension 40 can also be used to reference the location of the flexible casing with respect to a support, for example with respect to a printing plate-form. To this end, the tabs 42 could for example comprise two holes: one hole being mounted in a relief made on the printing plate-form for referencing its position and another hole received by the lower end of one post 38.

Advantageously, the sterile enclosure 2 can comprise a receiving sleeve 46 adapted to receive and hold the printing needle 6 before or after printing. In the embodiment illustrated on figure 1, the receiving sleeve 46 is integral with the side wall. The receiving sleeve 46 is preferably made with the same material as the flexible side wall.

This receiving sleeve may have an enough volume in order to receive and isolate bioink during the priming phase of the needle. For example, a volume comprised between 0.5 ml and 3 ml could be used.

Advantageously, the sterile enclosure 2 illustrated on figure 1 can comprise a needle protection cover 48 adapted to be snap on an abutment ring of the needle adapter. The needle protection cover 48 avoids the drilling of the flexible casing by the needle. According to a non-illustrated embodiment, the sterile enclosure comprises several needle adapters and printing needles. According to this embodiment, the flexible casing can for example have the shape of a glove. A needle adapter and a printing needle can be fixed at the end of each finger. According to this embodiment, at least the side wall is made with silicone.

Alternatively, the base wall 10 is rigid. For example, the base wall is made from a biocompatible plastic. The side wall is fixed to the edges of the rigid base wall in a sealed manner.

Referring to FIG. 2, the sterile enclosure 50 according to a second embodiment of the present invention is similar to the sterile enclosure 2 according to the first embodiment at the exception of the fact that this flexible casing presents a different shape and that the support armature is different. The technical elements of the second embodiment identical to the technical elements of the first embodiment have been referenced by the same references and will not be described again. Only the differences between the second embodiment and the first embodiment will be described in detail.

In this second embodiment, the sterile enclosure 50 comprises a flexible casing 52 having a cylinder shape in its lower part and a general flared shape in its upper part. The upper part 30 has for example the shape of a truncated cone or the shape of a truncated pyramid.

The support armature 54 comprises several support beams 56 extending along a vertical direction. The lower end of the support beams is provided with feet 58 to keep the beams vertical. The support beams 56 are regularly space around the flexible casing and are attached directly against the side wall 8.

Advantageously, the support beams 56 maintain the lower part 28 of the side wall in a vertical position. So, even when a printing syringe causes a lowering of the upper part 30 of the side wall, the side wall does not touch the printed three-dimensional biological structure which laid down on the interior face of the base wall.

In the illustrated embodiment, an internal face of an edge of the flexible side wall is fixed to the internal face of a peripheral edge of the base wall 10 to form an extension 40 which extends in a horizontal plane.

The extension surrounds the lower cylindrical part of the flexible casing 52. In the illustrated embodiment, the extension 40 does not comprise perforated tabs.

Alternatively, the extension comprises perforated tabs which are used to reference the location of the flexible casing with respect to a printing platform.

Alternatively, according to a non-illustrated embodiment, the sterile enclosure 50 comprises a rigid base stiff framework. The flexible film of the base wall is fixed to the rigid base stiff framework so that the film is stretched to form a flat surface. Alternatively, the base wall 10 is rigid. For example, the base wall is made from a biocompatible plastic. According to this alternative, the side wall is fixed to the edges of the rigid base wall in a sealed manner.

Referring to FIG. 3, the sterile enclosure 60 according to a third embodiment of the present invention is similar to the sterile enclosure 2 according to the first embodiment at the exception of the fact the flexible casing comprises a rigid frame 64 provided with a membrane 62 instead of a needle adapter 4. The technical elements of the third embodiment identical to the technical elements of the first embodiment have been referenced by the same references and will not be described again. Only the differences between the first embodiment and the third embodiment will be described in detail.

In the non-limiting embodiment illustrated on figure 3, the rigid frame 64 has a general oblong shape. It comprises a central aperture. The membrane 62 covers the central aperture in a sealed manner. The membrane is fixed to the interior face of the rigid frame. The membrane constitutes a septum that is intended to be punctured by a printing needle of a syringe or by a printing cartridge. The upper edge of the flexible side wall 8 is fixed to the outer periphery of the rigid frame.

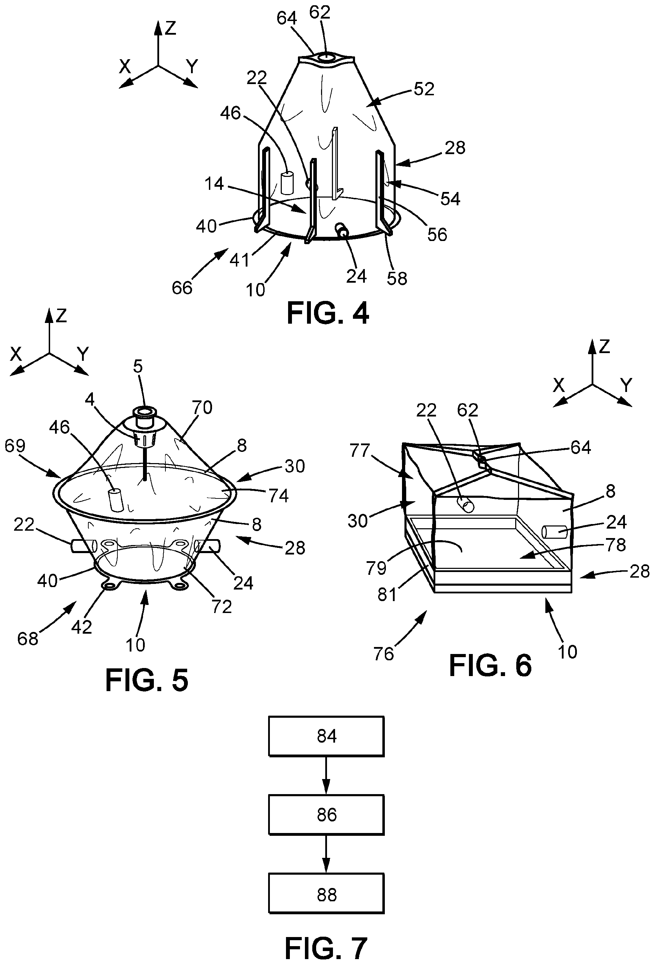

Referring to FIG. 4, the sterile enclosure 66 according to a fourth embodiment of the present invention is similar to the sterile enclosure 50 according to the second embodiment at the exception of the fact the sterile enclosure comprises a rigid frame 64 provided with a membrane 62 instead of a needle adapter 4. The technical elements of the fourth embodiment identical to the technical elements of the second embodiment have been referenced by the same references and will not be described again. Only the differences between the second embodiment and the fourth embodiment will be described in detail.

In the non-limiting embodiment illustrated on figure 4, the rigid frame 64 has a general oblong shape. It comprises a central aperture. The membrane 62 covers the central aperture in a sealed manner. The membrane is fixed to the interior face of the rigid frame. The membrane constitutes a septum that is intended to be punctured by a printing needle of a syringe or by a printing cartridge. The upper edge of the flexible side wall 8 is fixed to the outer periphery of the rigid frame.

The sterile enclosure 68 according to a fifth embodiment of the present invention comprises a flexible and hermetically closed casing 69 represented on figure 5 and a support armature not represented on figure 5.

The technical elements of the fifth embodiment identical to the technical elements of the first embodiment have been referenced by the same references and will not be described again.

The flexible casing 69 is made from a first sheet 70 whose edges are welded to the edges of the second sheet 72. The sheets are made from a thermoplastic elastomer or a thermoset.

The first sheet 70 forms the base wall 10 and the lover part 28 of the side wall. the second sheet 72 constitutes the upper part 30 of the side wall and the top of the flexible casing.

The sterile enclosure 68 according to the fifth embodiment of the present invention comprises a needle adapter 4 fixed in an opening of the second sheet 72. The second sheet 72 is fixed around the needle adapter 4 in a sealed manner. The lower edge 74 of the second sheet is fixed to the first sheet 10 in a sealed manner, preferably by welding.

A printing needle 6 is fixed to the needle adapter 4.

The non-represented support armature is adapted to hold the lower part 28 of the flexible casing 69 so that the lower part 28 of the side wall will not come into contact with the interior face of the base wall 10 or with the printed three-dimensional biological structure deposited on it.

Alternatively, the sterile enclosure 68 does not comprise a support armature. In this case, the lower part 28 of the flexible casing 69 can be placed in a rigid receptacle. The latter one does not need to be sterilized.

In the illustrated embodiment, the first 70 and the second 72 sheets are circular. Alternatively, the first and the second sheets have a square or a rectangle shape. Advantageously, this flexible casing 69 is easy to manufacture.

Referring to FIG. 6, the sterile enclosure 76 according to a sixth embodiment of the present invention, comprises a partially flexible casing 77. The casing 77 comprises a rigid base wall 78, four flexible side walls 80 and a flexible top wall 82. The other technical elements of the sixth embodiment identical to the technical elements of the first embodiment have been referenced by the same references and will not be described again.

The rigid base wall 78 is for example made in plastic.

Advantageously, in the illustrated embodiment the base wall 78 comprises a plate 79 surrounded by vertically extending rims 81. The rims 81 form a rigid lower part 28 of the flexible casing. The flexible side wall 8 forms the upper part 30 of the flexible casing. In the illustrated embodiment, the rims 81 have a height corresponding to less than 20 % of the total height of the casing. Alternatively, the rims can have a height which represents 50 % of the total height of the casing.

The flexible side walls 80 and the top wall 82 is made of a thermoplastic elastomer sheet or thermoset sheet. The side walls and the top wall forms with the rigid base wall, an hermetically closed casing 77. This casing 77 is partially flexible since both the side walls 80 and the top wall 82 are flexible.

In the illustrated embodiment, the partially flexible casing 77 has a general shape of a parallelepiped. Other shape can also be used.

The top wall 82 is formed by welding a triangular portion of each side wall. Other shape can also be used.

The sterile enclosure 76 comprises a rigid frame 64 fitted with a membrane 62. The rigid frame 64 is fixed to the top wall in a sealed manner.

Alternatively, the rigid frame 64 and the membrane 62 is replaced by a needle adapter 4 and a printing needle held by the needle adapter.

Alternatively, the base wall 10 comprises only a flat rigid plate and a support armature 36 similar to the one described in relation with the first embodiment. The support armature is placed around the flexible casing. Attachment links 32 are fixed to the side walls 80 to prevent them from falling on the base wall 10.

Alternatively, the base wall 10 comprises a rigid plate and support beams 56 fixed to the rigid plate. In this embodiment, the flexible side wall 80 is integral with the support beams to keep the lower part 28 of the flexible casing at distance from the base wall 10.

All alternative embodiments described in connection with the first and second embodiments also apply to the other embodiments. They have not been described again to simplify the description.

This invention also concerns a method of printing and culturing a three-dimensional biological structure in a sterile enclosure.

When using a sterile enclosure as described in the first, second and fifth embodiments, a printing needle is fixed to the needle adapter 4 and the method begins with a step of fixing a printing syringe or a printing cartridge to the connector 5 of the needle adapter 4. When using a sterile enclosure as described in the third, fourth and sixth embodiments, the method begins with a step during which the membrane 62 forming the septum is punctured by a printing syringe.

Alternatively, the printing needle may be integral with the cartridge and the method begins with the filing of the cartridge with the bioink.

During a priming phase, the rate of flow of the printing needle is regulated.

During a step 84, a three- dimensional biological structure is printed or deposited on the base wall 10 of the flexible casing. The upper part 30 of the flexible casing 12 authorizes the movement of the printing needle 6 in a vertical direction Z, as well as in two perpendicular horizontal directions X, Y during the biological printing step while keeping the sterility of the interior chamber.

The step 84 is followed by a step 86 during which two opposite parts of the side wall 8 are pinched and fixed together along a horizontal plane to separate an upper portion of the interior chamber comprising the printing needle 6 from the inferior portion of the interior chamber to avoid a contamination of the printed three-dimensional structure by the residual bioink remaining in the needle. The fixing can be done with a clamp or by welding the two parts of the side wall together.

During a step 88, a culture media is introduced into the interior chamber for feeding the three- dimensional biological structure.

Alternatively, step 86 can be performed after step 88.

## Claims

1. Sterile enclosure (2,50,68) comprising:
- a base wall (10),
- at least one flexible side wall (8); the at least one side wall (8) forming with the base wall (10) an at least partially flexible casing (12, 52,69) delimiting an interior chamber (14),
- at least one fluid inlet port (22) and at least one fluid outlet port (24) which allow the circulation of a culture media, **characterized in that** it comprises a needle adapter (4) tightly fixed to the at least one flexible side wall (8), the needle adapter (4) being intended to hold a printing needle (6),
the at least partially flexible casing (12, 52,69) being intended to protect a biological three-dimensional structure against contamination and authorizing at the same time the move of the needle adapter (4) in at least two directions (X, Y) during a sterile printing of a biological three-dimensional structure in the interior chamber.

2. Sterile enclosure (60,66,76) comprising:
- a base wall (10),
- at least one flexible side wall (8); the at least one side wall (8) forming with the base wall (10) at least partially flexible casing (12, 52,77) delimiting an interior chamber (14),
- at least one fluid inlet port (22) and at least one fluid outlet port (24) which allow the circulation of a culture media, **characterized in that** it comprises a rigid frame (64) fixed tightly to the at least one flexible side wall (8) and a membrane (62) supported by the rigid frame, the membrane (62) forming a septum conform to be punctured by a printing needle (6),
the at least partially flexible casing (12, 52,77) being intended to protect a biological three-dimensional structure against contamination and authorizing at the same time the move of a needle in at least two directions (X, Y, Z) during a sterile printing of a biological three-dimensional-structure in the interior chamber.

3. Sterile enclosure (2,50,68; 60,66,76) according to any of claims 1 and 2, which comprises a support armature (26) holding a lower part (28) of the at least partially flexible casing (12, 52,69,77) to keep the lower part of the at least flexible side wall (8) away from the base wall (10).

4. Sterile enclosure (2,50,68; 60,66,76) according to any of claims 1 to 3, wherein an internal face of an edge of the flexible side wall is sealed to the internal face of a peripheral edge of the base wall (10) to form an extension (40) extending in a horizontal plane.

5. Sterile enclosure (2,68; 60,76) according to any of the claims 3 and 4, wherein the support armature (26) is outside and spaced apart from the at least partially flexible casing (12,69,77), the sterile enclosure (2,68; 60,76) comprising attachment links (32) fixed to the at least one side wall (8) and to the support armature (26) to keep the lower part (28) of the at least flexible side wall at distance from the base wall (10).

6. Sterile enclosure (2,68; 60,76) according to any of claims 3 to 5, wherein the support armature (26) comprises a frame (36) and posts (38) fixed to the frame, the frame (36) encircling the at least partially flexible casing (12,69,77).

7. Sterile enclosure (2,68; 60,76) according to the combination of claims 4 and 6, wherein the at least one extension (40) comprises through holes (44) and wherein the post (38) of the support armature (26) are positioned into the through holes (44).

8. Sterile enclosure (50,66,76) according to claim 3, wherein the support armature (26) comprises at least one support beam (56) integral with the at least flexible side wall (8), the at least one support beam (56) keeping the lower part (28) of the at least partially flexible casing (52,77) at distance from the base wall (10).

9. Sterile enclosure (2,50,68; 60,66) according to any of claims 1 to 8, wherein the base wall (10) is flexible.

10. Sterile enclosure (68) according to any of claims 8 and 9, wherein the flexible casing (69) is made from a first sheet (70) welded to a second sheet (72); the first sheet (70) forming the upper part (30) of the flexible casing; the second sheet (72) forming the base wall (10) and the lower part of the flexible casing.

11. Sterile enclosure (76) according to any of claims 1 to 8 and 10, wherein the base wall (10) is rigid.

12. Sterile enclosure (2,50,68) according to any of claims 1, 3 to 11, wherein the at least one needle adapter (4) comprises at least one aseptic connector (5) conform to be connected to a printing cartridge in an aseptic manner.

13. Sterile enclosure (2,50,68) according to any of claims 1, 3 to 11, which comprises a printing cartridge integral with the at least one needle adapter (4).

14. Sterile enclosure (2,50,68; 60,66,76) according to any of claims 1 to 13, wherein the at least one side wall (8) is made of one material among a thermoplastic elastomer and a thermoset and a silicone.

15. Method of printing and culturing a three dimensional biological structure in a sterile enclosure conform to any of claims 1 to 14, a needle (6) being fixed to the needle adapter (4); the method comprising the successive following steps:
- printing (84) of a biological three-dimensional structure, the at least partially flexible casing (12,52,69,77) authorizing the move of the needle (6) in at least two directions (X Y, Z) during the biological printing step while keeping the sterility of the interior chamber (14),
- supplying (88) a culture media to the interior chamber (14).

16. Method according to claim 15, the method further comprising a step of pinching and fixing two opposite parts of the side wall (8) to isolate the needle (6) from the biological three-dimensional structure.
